# EUROPEAN PATENT SPECIFICATION

(11) **EP 3 503 798 B1**
(45) Date of publication and mention of the grant of the patent: **29.09.2021**
(21) Application number: 17772766.6
(22) Date of filing: 25.08.2017
(51) Int. Cl.: A61B 5/282, A61B 5/0531, A61B 5/296

(54) **MEDICAL DEVICE WITH ROTATIONAL FLEXIBLE ELECTRODES**
MEDIZINISCHE VORRICHTUNG MIT FLEXIBLEN DREHBAREN ELEKTRODEN
DISPOSITIF MÉDICAL AVEC ÉLECTRODES FLEXIBLES ROTATIVES

(30) Priority: 25.08.2016 PT 2016109596
(43) Date of publication of application: 03.07.2019
(73) Proprietor: INESC TEC - Instituto de Engenharia de Sistemas e Computadores, Tecnologia e Ciência, 4200-465 Porto (PT)
(72) Inventor: TRIGUEIROS DA SILVA CUNHA, João Paulo, 4200 - 465 Porto (PT)
(74) Representative: Patentree
(86) International application number: PCT/IB2017/055139
(87) International publication number: WO 2018/037389

(56) References cited:
- EP-A1- 1 782 729
- DE-U1- 20 301 410
- KR-A- 20120 068 264
- US-B1- 6 605 046

## Description

### Technical field

The present disclosure relates to a portable wearable medical device with two electrode connection points for simultaneous corporal attachment of two electrodes for electrocardiogram, ECG or EKG, monitoring.

### Background

Document WO2014107018A1 discloses a patch-type electrode for measuring bio signals with a main body detachable structure and a plurality of electrode portions provided on periphery portions of a fabric layer with a plurality of snap buttons formed at the end of signal lines connecting with said electrodes.

The adhesive layer of WO2014107018A1 is adhesively attached to the human body such that body movement, in particular movement stretching or shrinking the area between the electrodes, makes its use uncomfortable because the adhesive layer is limited by its elasticity capabilities (and of the signal lines).

Document US6327487B1 discloses an electrode assembly for ECG monitoring. A bioelectric interface includes an adhesive sheet in functional combination with a support sheet. The electrodes are affixed to the support sheet in a manner such that their relative positions with respect to one another are essentially fixed.

The adhesive and support sheets of US6327487B1 are adhesively attached to the human body such that body movement, in particular movement stretching or shrinking the area between the electrodes, makes its use uncomfortable because the sheets are limited by their elasticity capabilities.

Document CN104352237A discloses an electrocardiogram detection device comprising a plug board and a junction box from which one or more lead electrodes are connected. Each electrode has a flat wire and a body suitable to be fixed on the back of a patient for a long time without adverse impact on the patient.

The device of CN104352237A is left dangling from the patient skin, thus making its use uncomfortable, especially during walking or running, because the lead electrode swings on its wire part about the fixed part to the patient skin. Further medical devices with electrodes for acquiring ECG signals are for instance described in US6605046B1, DE20301410U1, KR20120068264A and EP1782729A1.

These facts are disclosed in order to illustrate the technical problem addressed by the present disclosure.

### General Description

It is disclosed a medical device comprising a main body and only two electrodes for acquiring an ECG signal,
wherein each electrode comprises a base which has an adhesive surface for adhering and electrically contacting to a medical subject and one of said electrodes comprises an arm which projects from the base,
the arm being attached, at one end, to said base and, at the other end, to an electrode attachment of the main body,
wherein said arm is both, rotatably attached about the respective base and about the respective electrode attachment of the main body such that the distance between the two electrode bases is variable when attached to the medical subject.

In an embodiment, each of both electrodes comprises an arm which projects from the respective base, each said arm being attached, at one end, to the respective base and, at the other end, to a respective electrode attachment of the main body, wherein each said arm is rotatable about the respective base and about the respective electrode attachment of the main body such that the distance between the two electrode bases is variable.

In an embodiment, said arm is flexible.

In an embodiment, the arm is rotatable such that the distance between the two electrode bases is variable by breathing of the subject.

In an embodiment, said arm is an electrically insulating flexible tab which comprises an electrically conductive cable for connecting between the electrode base and the electrode attachment of the main body, the tab and cable being arranged such that the cable is electrically insulated from contact with the subject body.

In an embodiment, said arm comprises a conductive core mounted between two flat non-conductive materials.

In an embodiment, the conductive core is made of a flat laminar metallic wire, a twisted multi-filament wire or a micro-coaxial, core and shield separated by an insulator, cable.

In an embodiment, the flat non-conductive materials are made of polymer, fabric, cellulose or combinations thereof.

In an embodiment, the rotatable attachment of the arm comprises a clamp rotatable about a round electrical terminal.

In an embodiment, the arm is comprised of a first arm section and a second arm section, and the two arm sections are rotatably articulated between themselves.

In an embodiment, the electrode attachments to the main body of the device are located in a surface of said device along a rectilinear line of said surface that comprises the projection of the device centre of mass projected perpendicularly to said surface.

In an embodiment, the electrode attachments to the main body of the device are located in a surface of said device at the middle of the device height.

In an embodiment, the electrode attachments to the main body of the device are located in a surface of said device symmetrically from a bisecting plane of said device.

In any of the above mentioned configurations, the disclosed device is able to take advantage of the rotational coupling for reducing signal noise. It is preferably used a natural flexibility of the embodiment material (e.g. polymer) to add tolerance to movement incorporated by the rotational contact (one or two, depending on the configuration). This results into a joint noise reduction characteristic in the disclosed device and simplifies its manufacturing process and price. This flexibility is particularly important when the electrode arms are either rotatably attached about the respective base or are rotatably attached about the respective electrode attachment, i.e. not both rotatably attached about the respective base and also about the respective electrode attachment.

### Brief Description of the Drawings

The following figures provide preferred embodiments for illustrating the description and should not be seen as limiting the scope of invention.
**Figure 1****:** Schematic representation of an embodiment of the overall design and its attachment to the human body for biosignal collection.
**Figure 2****:** Schematic representation of an embodiment with "rotational" electrodes.
**Figure 3****:** Schematic representation of an embodiment with "rotational" electrodes comprising a base and an arm which is rotatably attached about the respective base. The rotation feature is illustrated.
**Figure 4****:** Schematic representation of an embodiment with "rotational" electrodes comprising a base and an arm which is rotatably attached about an electrode attachment arm.
**Figure 5****:** Schematic representation of an embodiment with "rotational" electrodes comprising a base and an arm which is both rotatably attached about the respective base and about an electrode attachment to the main body of the device. The rotational feature is illustrated.
**Figure 6****:** Schematic representation of an embodiment with "rotational" electrodes wherein the electrode arms are placed such that the electrode attachment to the main body is placed upwards in reference to the electrode base where the rotational articulation is placed. The rotational feature is illustrated when horizontal stretching forces are applied.
**Figure 7****:** Schematic representation of an embodiment with "rotational" electrodes comprising a base and an arm, wherein the electrode arm is an insulating tab comprising a conductive core.
**Figure 8****:** Center of mass of the device and possible "anchor" positions for the attachment arm are presented at the top of the figure. Below, a schematic representation of an embodiment with "rotational" electrodes comprising a base and an arm, wherein the electrode attachments to the main body of the device are located in one of the "anchor" points of the device (1/2 vertical, 1/8 horizontal).
**Figure 9****:** Schematic representation of an embodiment with "rotational" electrodes comprising a base and an arm, wherein the electrode attachments to the main body of the device are located at the middle of the device height and in the middle of half of the device length, wherein the device height is measured in respect of the vertical of its placement adhering to a medical subject. This configuration allows to cover less horizontal length of the body surface that may be useful in some usage situations.
**Figure 10****:** Schematic representation of a different form-factor (non-rectangular) embodiment with one "rotational" electrode and one fixed electrode with corresponding illustration of the rotational effect.

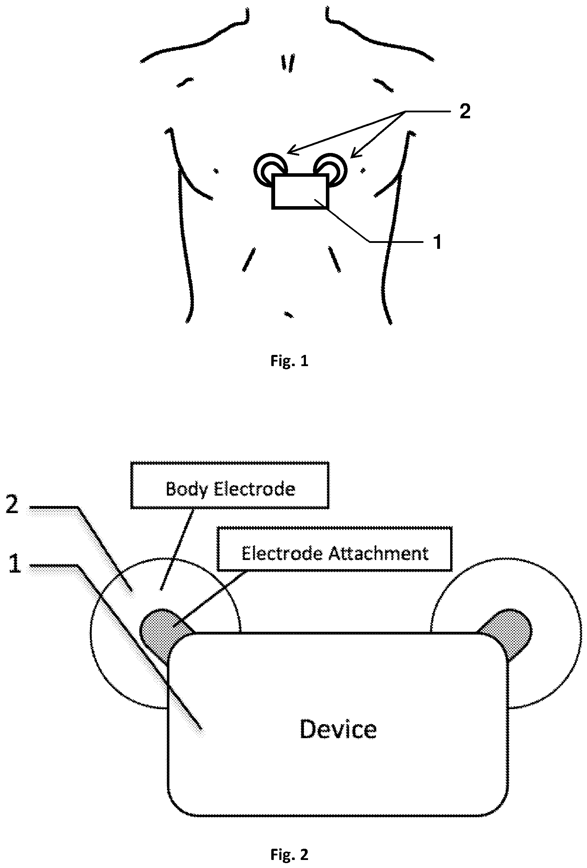

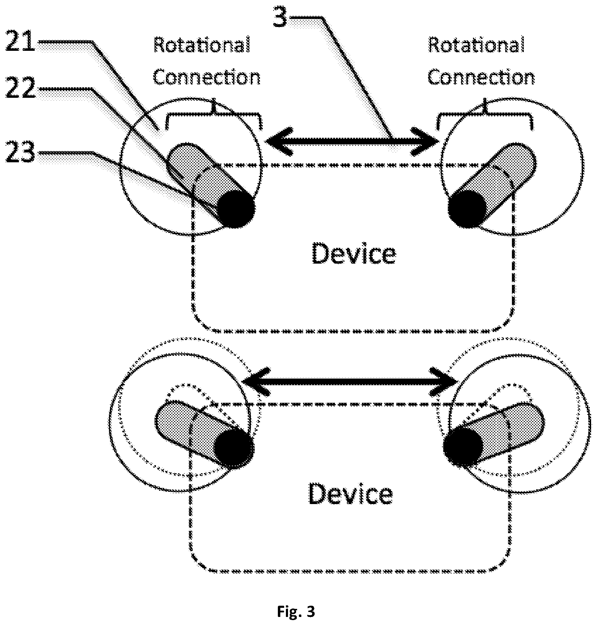

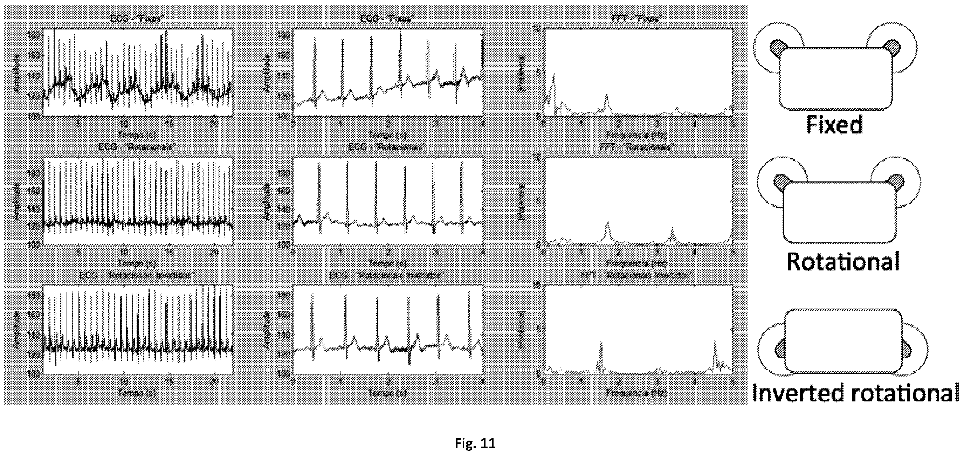

**Figure 11****:** Results of ECG tracings obtained in a test of three versions of the disclosed device with electrodes connection in the upper part of the device (Fig. 2, 3). The first column shows collected ECG, the second column features a zoom of the same signal, and in the third column a spectral analysis is presented. The images in the first row depict the test with the disclosed device with non-rotational ("fixed") electrodes, the second line depicts disclosed device with "rotational" electrodes and the third line represents the disclosed device in an inverted "rotational" electrodes configuration.
**Figure 12****:** Results of ECG tracings obtained in a test of two versions of the disclosed device with electrodes connections according to Fig. 8 with different attachment arms: one stiff and another flexible. The first column shows collected ECG, the second column featured a zoom of the same signal; the third column presents a spectral analysis. The first line of images shows results with the configuration with stiff attachment arm, and the second line shows configuration with flexible attachment arm.
**Figure 13****:** Results of ECG tracings obtained in a test of two versions of the disclosed device with electrodes connections according to Fig. 8. The first column shows collected ECG, the second column features a zoom of the same signal; the third column presents a spectral analysis. The first line of images shows the results of the configuration with "fixed" electrodes, and the second line shows configuration with "rotational" electrodes. Both configurations used flexible attachment arms.

### Detailed Description

It is disclosed a wearable ambulatory biosignal monitoring device capable of using a combination of rotational and flexible attachment arms that minimize signal noise induced by movement, largely improving the signal-to-noise ratio of the resulting acquired signal. A specific profiling device family (with multiple combinations of the disclosed features) for acquiring ECG signal of 1-lead with only two electrodes, without requiring a third reference electrode, was developed to demonstrate the disclosure. The disclosure, also as depicted in the attached drawings, includes using the electrodes as the device support, being placed on the thorax region as an example of the disclosure applied to a specific biomedical device shown in **Figure 1****.** In this Figure, a device or main body 1 is connected to two electrodes 2 that are attached to the subject. The disclosed device can be also adapted to collect other biosignals such as Electromyography (EMG), Skin Conductance Level (SCL), etc.

The disclosure uses rotational connectors leading to a reduction of the noise induced by mechanical tractions that electrode can be subjected. This noise is caused particularly by the stretching motion of the body, for example by breathing movements, and the effects of gravity on the device when the user is in movement (e.g. walking, running, etc.). In this way, the rotational joint in each one of the "arms" connecting the electrodes allows a slight articulatory rotation of the electrodes when, for example, the user inhales and exhales. This is an improvement over other devices that, for example, stress the skin because they cannot follow the breathing movement, or transmit the vibrations of running motions and inertia of the device directly to the skin-electrode contact, thus causing signal noise that degrades the quality of the targeted signal.

**Figure 2** shows a schematic representation of an embodiment with "rotational" electrodes **2** (body and attachment) coupled to a device main body **1.**

**Figure 3** shows a schematic representation of an embodiment with "rotational" electrodes comprising a base (21) for adhering and contacting to the subject body and an arm (22) which is attached to said base and rotatably attached about a respective arm attachment (23) to the main body of the device. The rotation feature is illustrated when horizontal stretching is applied (3). There is a small amount of torsion at the arm 22 which can be accommodated by having the arm and/or the base made of a flexible material.

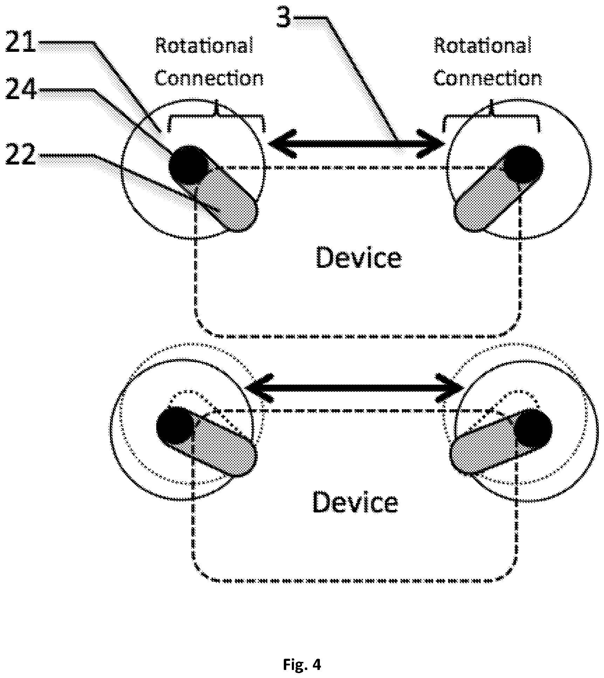

**Figure 4** shows a schematic representation of an alternative embodiment with "rotational" electrodes comprising a base (21) for adhering and contacting to the subject body and an arm (22) which is rotatably attached about the respective base (24) and is also attached to the main body of the device. The rotation feature is illustrated when horizontal stretching is applied (3). There is a small amount of torsion at the arm 22 which can be accommodated by having the arm and/or the base made of a flexible material.

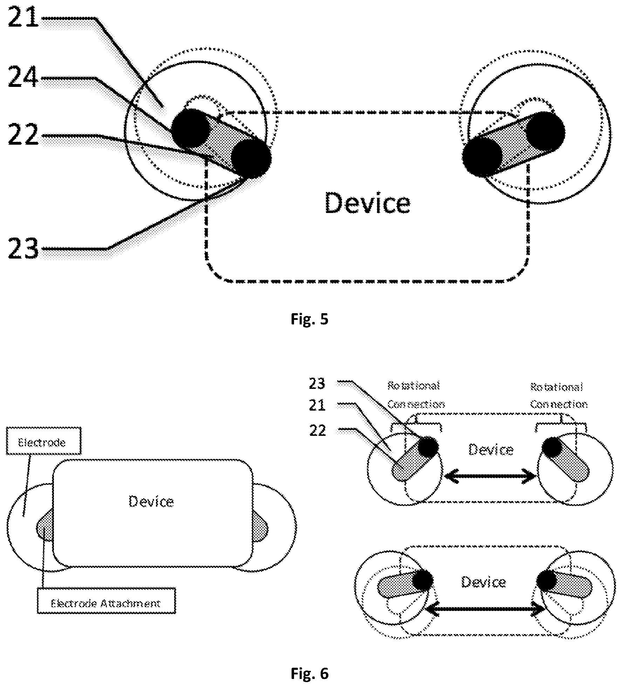

**Figure 5** shows a schematic representation of an embodiment with "rotational" electrodes comprising a base (21) for adhering and contacting to the subject body and an arm (22) which is both rotatably attached about the respective base (24) and about an electrode attachment (23) to the main body of the device. The rotation feature is illustrated when horizontal stretching is applied (3).

The fact that the rotational joints are displaced gives greater stability to the electrode because they are not submitted to any pressure during stretching and other movements such as breathing. This effect is reflected in an ECG tracings with a more defined and stable baseline and lower high-frequency interference, as it will be seen ahead.

The main feature of this disclosed design is the fact that it has a rotational connection between the device and the electrode. This means that the electrode centroid and the electrode connection centroid in the device are not the same, enabling to create an articulation of rotational nature between the device and the electrode materialized as an attachment arm. This results in a direct improvement in the ECG waveform quality, making it more immune to motion noise, improving largely the signal-to-noise ratio of the collected waveform, in particular the baseline along the signal. This design prevents, for example, the noise effects of skin stretching during chest respiration movements. Furthermore, other movements of the device and electrodes as result of subject activities such as walking, running, etc. are also attenuated by the rotational effect of the disclosure.

The results of several tests performed with different versions of the disclosed device are presented in Figures 11. It shows ECG tracings obtained in a test of three versions of the disclosed device with electrodes attachment arms in the upper part of the device, as described in the previous section. Baseline oscillations and some high-frequency noise are clearly more present in the first "fixed" configuration than in the other two versions.

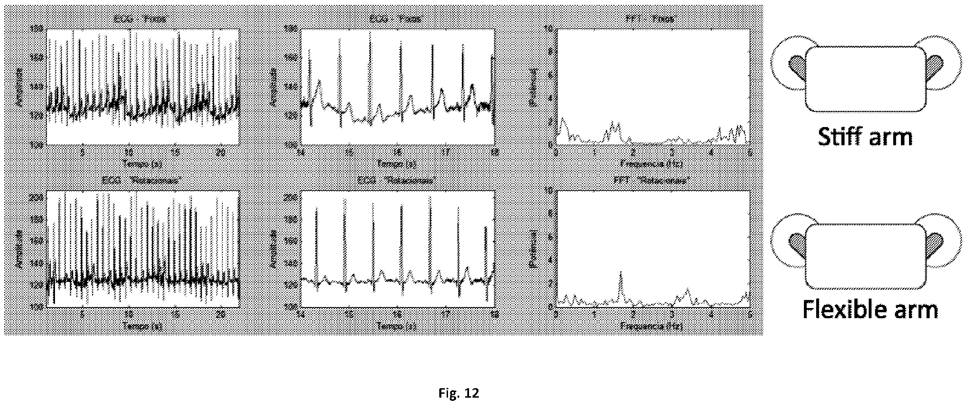

The second feature of the disclosure is the flexibility of its articulation arm which also helps in the collected biosignal quality. If the articulation is stiff it will pass all the motion forces to the electrode contact, inserting high noise levels into the collected signal. A flexible arm results in a "motion dumping" effect that is added to a smoother rotational movement contributing to a higher quality resulting waveform. Figure 12 shows in the first line of images an example of a rotational electrode design with a stiff arm attachment between the device and the electrodes base and in the second line an example of the same device with a rotational connection with flexible arm attachment between the device and the electrodes. The "stiff" attachment device presents larger baseline oscillations (low frequency) and high frequency noise component than the second "flexible" one, demonstrating clearly the effect of a flexible arm.

The third feature of the present disclosure is the articulation attachment variants that benefit from the disclosure. All the variants that include one or two rotational connections (as state above) between the device and the electrodes are included in this design concept. For example: the electrodes attachment in the device can be at any place of the main body; between the electrode and the device it can exist or not an extension made of all kind of material, preferably if they have flexibility characteristics as shown above; the electrode properties as for example size, shape and type (dry or wet) can be of any kind because motion noise is always a major problem in signal collection systems. The present disclosure minimizes this type of noise, resulting in a better signal-to-noise ratio of the collected signal. Figures 3, 4, 5, 6, 8, 9 and 10 illustrate several possible configurations of this third feature of the disclosure.

Different position of the electrodes, such as inverted "rotational" electrodes or attachment arms, such as more or less flexible, larger or smaller or articulated are also possible to obtain the desired effect. A test was also performed with the configuration shown in the Figure 6, wherein the rotational electrode attachments arms are inverted facing upwards in reference to the electrode base where the rotational articulation is placed. The rotational effect when horizontal stretching is applied is illustrated. As referred before, the results are also better than the non-rotational version of the device (Figure 11).

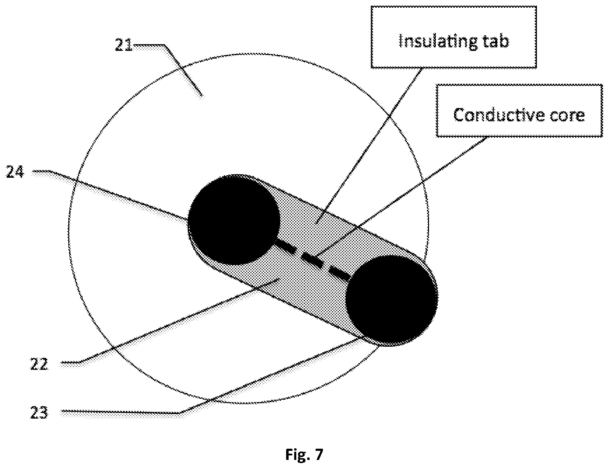

Electrode attachment arms may have different type of connection between the electrodes and the device, optionally having a rotational at one or the two ends. Furthermore, these attachment arms are composed of an insulator tab that embeds a conductive core, as depicted in Figure 7. The insulator tab may be made of polymer, fabric, cellulose or other insulator material or even combinations thereof, that covers the conductive core, for example, in a "sandwich" configuration. This conductive core can be a flat laminar metallic wire, a twisted multi-filament wire or a micro-coaxial, core and shield separated by an insulator, cable. Other configurations of this electrode component may be assembled to reach the same features herein disclosed.

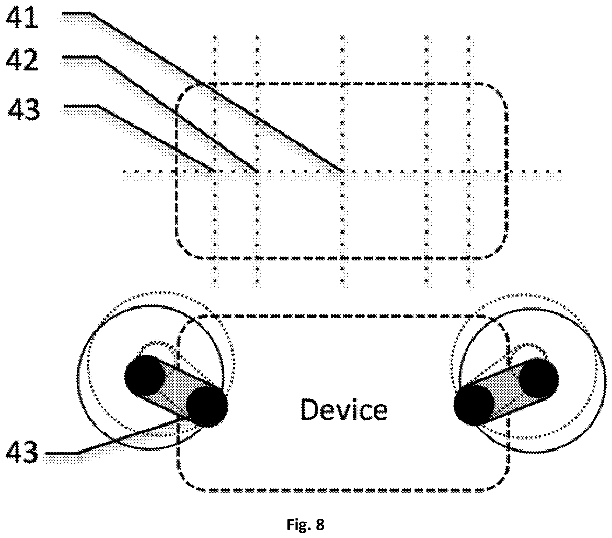

Figure 8 shows the center of mass of the device 41 and possible "anchor" positions for the attachment arm 42, 43 and a configuration with two rotational ends of the electrode attachment arm connected to "anchor" 43 (1/2 vertical, 1/8 horizontal). These options may dump better the device oscillation making it smaller by achieving a better mass distribution among the supporting contacts.

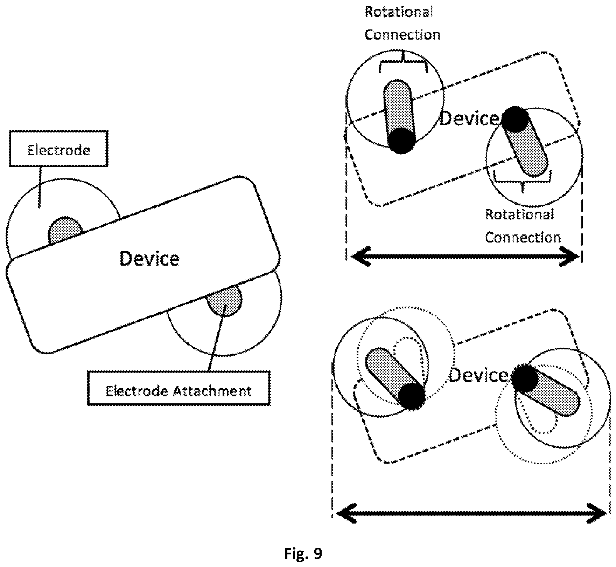

Figure 9 shows an example of the third feature of the disclosure where one rotational attachment arm is attached to the device in a vertical alignment. This configuration allows covering less horizontal length of the body surface that may be useful in some usage situations.

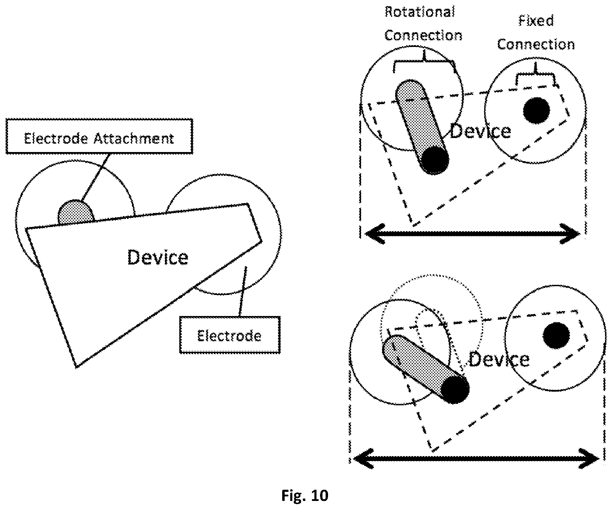

Figure 10 presents an example of a non-rectangular device with a fixed and a rotational electrode configuration, showing that the features disclosed in the present disclosure are not limited by the device form-factor and may also be used on only one of two (or possibly more) electrodes. Optionally, the second (fixed connection) electrode may also rotate about its attachment to the main device body.

A set of tests with different configurations were performed on a subject with device positioned as depicted in Figure 1, in a sitting position, with a constant inhale and exhale movement. For each of these tests, high quality ECG (500 Hz sampling frequency, no filter) epochs of ^{∼}20 seconds were collected and power spectrum estimation was computed using a Fast Fourier Transform (FFT) to quantify the frequency spectrum.

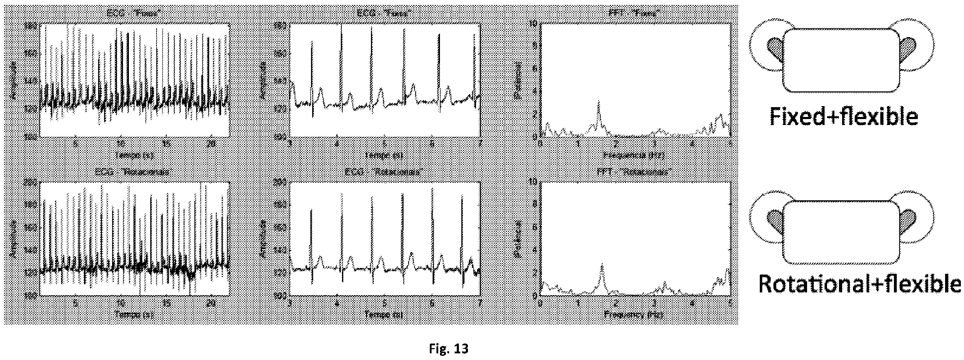

As can be seen by the results shown in Figures 11, 12 and 13, the rotational electrodes stabilize the baseline of the ECG trace, removing the oscillatory motion caused by breathing. The spectrum (FFT) demonstrates the same: only in the "fixed" electrodes exists a high peak at low frequencies (≈0.2 Hz) due to the movement. In all rotational electrodes configuration during the movement of inhaling and exhaling some high frequency noise is present but highly reduced in amplitude when compared with non-rotational ("fixed") configurations, as confirmed in the ECG segments in the middle column of the corresponding figures. As the waveform is not modified with the rotation noise providing good electrical contact that can be achieved by stainless steal, gold-platted metal or any other good conducting robust material, it is possible to conclude that the noise caused by the rotation of the electrodes does not significantly affect the ECG trace.

Stiff and flexible attachment arms also present differences, being the flexible feature of the disclosed device a characteristic that improves signal-to-noise ratio as shown in Figure 12.

In terms of comfort, the participant stated that with the rotational and flexible configurations do not feel the skin stretching he felt with the non-rotational configurations. This confirms, qualitatively, a better comfort factor of the disclosed device devices than others.

The disclosure should not be seen in any way restricted to the embodiments described and a person with ordinary skill in the art will foresee many possibilities to modifications thereof.

The above described embodiments are combinable.

The invention is defined in the appended claims.

## Claims

1. Medical device comprising a main body (1) and only two electrodes (2) for acquiring an ECG signal,
wherein each electrode (2) comprises a base (21) which has an adhesive surface for adhering and electrically contacting to a medical subject and one of said electrodes (2) comprises an arm (22) which projects from the base (21), the arm (22) being attached, at one end, to the respective electrode base (21) and, at the other end, to an electrode attachment of the main body (1),
wherein said arm (22) is both, rotatably attached about the respective base and about the respective electrode attachment of the main body (1), such that the distance between the two electrode bases (21) is variable when attached to the medica subject.

2. Device according to claim 1 wherein each of the two electrodes (2) comprises an arm (22) which projects from the respective base (21), each said arm (22) being attached, at one end, to the respective base (21) and, at the other end, to a respective electrode attachment of the main body (1), wherein each said arm (22) is rotatable about the respective base and about the respective electrode attachment of the main body (1) such that the distance between the two electrode bases (21) is variable.

3. Device according to any of the previous claims wherein said arm (22) is flexible.

4. Device according to any of the previous claims wherein the arm (22) is rotatable such that the distance between the two electrode bases (21) can be varied by the thorax breathing motion of the subject.

5. Device according to any of the previous claims wherein said arm (22) is an electrically insulating flexible tab which comprises an electrically conductive cable for connecting between the electrode base (21) and the electrode attachment of the main body (1), the tab and cable being arranged such that the cable is electrically insulated from contact with the subject body.

6. Device according to any of the previous claims wherein said arm (22) comprises a conductive core mounted between two flat non-conductive materials.

7. Device according to the previous claim wherein the conductive core is made of a flat laminar metallic wire, a twisted multi-filament wire or a micro-coaxial, core and shield separated by an insulator, cable.

8. Device according to claim 6 or 7 wherein the flat non-conductive materials are made of polymer, fabric, cellulose or combinations thereof.

9. Device according to any of the previous claims wherein the rotatable attachment of the arm (22) comprises a clamp rotatable about a round electrical terminal.

10. Device according to any of the previous claims wherein the arm (22) is comprised of a first arm section and a second arm section, and the two arm sections are rotatably articulated between themselves.

11. Device according to any of the previous claims wherein the electrode attachments to the main body (1) of the device are located in a surface of said device along a rectilinear line of said surface that comprises the projection of the device centre of mass projected perpendicularly to said surface.

12. Device according to any of the previous claims wherein the electrode attachments to the main body (1) of the device are located in a surface of said device at the middle of the device height.

13. Device according to any of the previous claims wherein the electrode attachments to the main body (1) of the device are located in a surface of said device symmetrically from a bisecting plane of said device.

## Patentansprüche

1. Medizinische Vorrichtung mit einem Hauptkörper (1) und nur zwei Elektroden (2) zur Erfassung eines EKG-Signals,
wobei jede Elektrode (2) eine Basis (21) mit einer Klebefläche zur Befestigung und Herstellung eines elektronischen Kontakts an einem Patienten umfasst und eine der genannten Elektroden (2) einen von der Basis (21) abstehenden Arm (22) umfasst,
wobei der Arm (22) an einem Ende an die entsprechende Elektrodenbasis (21) und, an dem anderen Ende, an eine Elektrodenhalterung des Hauptkörpers (1) befestigt ist, wobei der genannte Arm (22) sowohl drehbar an der entsprechenden Basis und der entsprechenden Elektrodenhalterung des Hauptkörpers (1) befestigt ist, sodass der Abstand zwischen den beiden Elektrodenbasen (21), wenn am Patienten befestigt, variabel ist.

2. Vorrichtung nach Anspruch 1, wobei jede der beiden Elektroden (2) einen Arm (22) umfasst, der von der entsprechenden Basis (21) absteht, wobei jeder genannte Arm (22) an einem Ende an der jeweiligen Basis (21) und am anderen Ende an einer entsprechenden Elektrodenhalterung des Hauptkörpers (1) befestigt ist, wobei jeder genannte Arm (22) um die entsprechende Basis und um die entsprechende Elektrodenhalterung am Hauptkörper (1) drehbar ist, sodass der Abstand zwischen (21) den beiden Elektrodenbasen (21) variabel ist.

3. Vorrichtung nach einem der vorangehenden Ansprüche, wobei der genannte Arm (22) flexibel ist.

4. Vorrichtung nach einem der vorangehenden Ansprüche, wobei der Arm (22) drehbar ist, so dass der sich Abstand zwischen den beiden Elektrodenbasen (21) durch die Atmungsbewegung des Thorax des Patienten verändern lässt.

5. Vorrichtung nach einem der vorangehenden Ansprüche, wobei der genannte Arm (22) ein elektrisch isolierender, flexibler Streifen ist, der ein elektrisch leitendes Kabel zur Verbindung zwischen der Elektrodenbasis (21) und der Elektrodenhalterung des Hauptkörpers (1) aufweist, wobei der Streifen und das Kabel so angeordnet sind, dass das Kabel gegen einen elektrischen Kontakt mit dem Körper des Patienten isoliert ist.

6. Vorrichtung nach einem der vorangehenden Ansprüche, wobei der genannte Arm (22) einen leitenden Kern umfasst, der zwischen zwei flachen, nichtleitenden Materialien angebracht ist.

7. Vorrichtung nach dem vorangehenden Anspruch, wobei der leitende Kern aus einem flachen, flächigen Metalldraht, einem verdrillten Multifilamentdraht oder einem Mikrokoaxialkabel, bei dem Kern und Abschirmung durch einen Isolator getrennt sind, besteht.

8. Vorrichtung nach Anspruch 6 oder 7, wobei die flachen nichtleitenden Materialien aus Polymer, Gewebe, Zellulose oder Kombinationen davon bestehen.

9. Vorrichtung nach einem der vorangehenden Ansprüche, wobei die drehbare Befestigung des Arms (22) eine um einen runden elektrischen Anschluss drehbare Klemme umfasst.

10. Vorrichtung nach einem der vorangehenden Ansprüche, wobei der Arm (22) aus einem ersten Armabschnitt und einem zweiten Armabschnitt besteht und die beiden Armabschnitte drehbar miteinander verbunden sind.

11. Vorrichtung nach einem der vorangehenden Ansprüche, wobei die Elektrodenhalterungen an dem Hauptkörper (1) der Vorrichtung in einer Oberfläche der genannten Vorrichtung entlang einer geraden Linie auf der genannten Oberfläche angeordnet sind, die den vorstehenden Teil des Masseschwerpunkts der Vorrichtung senkrecht zu der genannten Oberfläche umfasst.

12. Vorrichtung nach einem der vorangehenden Ansprüche, wobei die Elektrodenhalterungen an dem Hauptkörper (1) der Vorrichtung in einer Oberfläche der genannten Vorrichtung in der Mitte der Höhe der Vorrichtung angeordnet sind.

13. Vorrichtung nach einem der vorangehenden Ansprüche, wobei die Elektrodenhalterungen an dem Hauptkörper (1) der Vorrichtung symmetrisch zu einer halbierten Fläche der genannten Vorrichtung angeordnet sind.

## Revendications

1. Dispositif médical comprenant un corps principal (1) et seulement deux électrodes (2) pour acquérir un signal ECG,
dans lequel chaque électrode (2) comprend une base (21) qui a une surface adhésive pour adhérer et entrer électriquement en contact avec un sujet médical et une desdites électrodes (2) comprend un bras (22) qui émane de la base (21),
le bras (22) étant attaché, par une extrémité, à la base d'électrode respective (21) et, par l'autre extrémité, à une fixation d'électrode du corps principal (1),
dans lequel ledit bras (22) est à la fois fixé de façon pivotante autour de la base respective et autour de la fixation d'électrode respective du corps principal (1) tel que la distance entre les deux bases d'électrodes (21) soit variable lorsqu'elles sont attachées au sujet médical.

2. Dispositif selon la revendication 1, dans lequel chacune des deux électrodes (2) comprend un bras (22) qui émane de la base respective (21), chacun desdits bras (22) étant attaché, par une extrémité, à la base respective (21) et, par l'autre extrémité, à une fixation d'électrode respective du corps principal (1), dans lequel chacun desdits bras (22) est pivotable autour de la base respective et autour de la fixation d'électrode respective du corps principal (1) tel que la distance entre les deux bases d'électrodes (21) soit variable.

3. Dispositif selon l'une quelconque des revendications précédentes, dans lequel ledit bras (22) est flexible.

4. Dispositif selon l'une quelconque des revendications précédentes, dans lequel le bras (22) est pivotable tel que la distance entre les deux bases d'électrodes (21) puisse varier en fonction du mouvement respiratoire du thorax du sujet.

5. Dispositif selon l'une quelconque des revendications précédentes, dans lequel ledit bras (22) est une languette flexible électriquement isolante qui comprend un câble électriquement conducteur pour la connexion entre la base d'électrode (21) et la fixation d'électrode du corps principal (1), la languette et le câble étant arrangés tels que le câble soit électriquement isolé du contact avec le corps du sujet.

6. Dispositif selon l'une quelconque des revendications précédentes, dans lequel ledit bras (22) comprend un noyau conducteur monté entre deux matériaux non-conducteurs plats.

7. Dispositif selon la revendication précédente, dans lequel le noyau conducteur est constitué d'un fil métallique laminaire plat, d'un fil multifilaments torsadé ou d'un câble micro-axial, avec noyau et bouclier séparés par un isolant.

8. Dispositif selon la revendication 6 ou 7, dans lequel les matériaux non-conducteurs plats sont constitués de polymère, de tissu, de cellulose ou des combinaisons de ceux-ci.

9. Dispositif selon l'une quelconque des revendications précédentes, dans lequel la fixation pivotable du bras (22) comprend une pince pivotable autour d'un terminal électrique rond.

10. Dispositif selon l'une quelconque des revendications précédentes, dans lequel le bras (22) est constitué d'une première section de bras et d'une seconde section de bras, et les deux sections de bras sont articulées de manière pivotante entre elles.

11. Dispositif selon l'une quelconque des revendications précédentes, dans lequel les fixations d'électrode au corps principal (1) du dispositif sont situées dans une surface dudit dispositif le long d'une ligne rectiligne de ladite surface qui comprend la projection du centre de masse du dispositif projeté perpendiculairement à ladite surface.

12. Dispositif selon l'une quelconque des revendications précédentes, dans lequel les fixations d'électrode au corps principal (1) du dispositif sont situées dans une surface dudit dispositif au centre de la hauteur du dispositif.

13. Dispositif selon l'une quelconque des revendications précédentes, dans lequel les fixations d'électrode au corps principal (1) du dispositif sont situées dans une surface dudit dispositif symétriquement à un plan bissecteur dudit dispositif.
